# EUROPEAN PATENT APPLICATION

(11) **EP 1 944 057 A1**
(43) Date of publication of application: **16.07.2008**
(21) Application number: 06811033.7
(22) Date of filing: 02.10.2006
(51) Int. Cl.: A61N 1/30

(54) **IONTOPHORESIS APPARATUS CAPABLE OF CONTROLLING DOSE AND TIMING OF ADMINISTRATION OF SLEEP INDUCER AND ANALEPTIC AGENT**

(30) Priority: 30.09.2005 JP 2005287248
(71) Applicant: TTI ELLEBEAU, INC., Tokyo 140-0002 (JP)
(72) Inventor: AKIYAMA, Hidero c/o TTI ellebeau, Inc. Shinkan Building, Tokyo 140-0022 (JP); NAKAYAMA, Mizuo c/o TTI ellebeau, Inc. Shinkan Building, Tokyo 140-0022 (JP); MATSUMURA, Takehiko c/o TTI ellebeau, Inc. Shinkan Building, Tokyo 140-0022 (JP); MATSUMURA, Akihiko c/o TTI ellebeau, Inc. Shinkan Building, Tokyo 140-0022 (JP)
(74) Representative: Liesegang, Eva
(86) International application number: PCT/JP2006/319685
(87) International publication number: WO 2007/037476

(57) **Abstract**

The present invention relates to an iontophoresis device capable of controlling the administered dose and the timing of dose of a sleep-inducing agent and a stimulant individually. More specifically, the present invention relates to the iontophoresis device comprising: an electric power source device; a drug administration means comprising at least two or more electrode assemblies each comprising an ionic drug, the drug administration means being connected to the electric power source device; and a current control means for controlling a current flowing to the electrode assemblies individually, wherein the current flowing from the current control means allows the electrode assemblies to release the ionic drug in a predetermined amount at a predetermined time so that the ionic drug is administered transdermally to a living body, and wherein at least one of the two or more electrode assemblies comprising the ionic drug comprises a sleep-inducing agent as the ionic drug, and at least another one of the two or more electrode assemblies comprising the ionic drug comprises a stimulant as the ionic drug.

## Description

### CROSS-REFERENCE TO RELATED APPLICATIONS

This application claims the benefit of priority to the Japanese Patent Application No. 2005-287248 (filed on date: September 30, 2005), the entire disclosure of which is incorporated herein by reference.

### BACKGROUND OF THE INVENTION

### Field of Invention

The present invention relates to a technique (transdermal drug delivery) for transdermally administering various ionic drugs by means of iontophoresis. More specifically, the present invention relates to an iontophoresis device for administering a sleep-inducing agent and a stimulant to a living body while controlling the administered dose and the timing of dose thereof individually.

### Background Art

A method of introducing (permeating) an ionic drug placed on the surface of the skin or mucosa (hereinafter, merely referred to as "skin") of a predetermined site of a living body into the body through the skin by giving the skin an electromotive force sufficient to drive the ionic drug is called iontophoresis (iontophorese, ion introduction method, ion permeation therapy) (see, for example, JP 63-35266 A).

For example, positively charged ions are driven (transported) into the skin on the side of an anode (positive electrode) in an electric system of an iontophoresis device. On the other hand, negatively charged ions are driven (transported) into the skin on the side of a cathode (negative electrode) in the electric system of the iontophoresis device.

Conventionally, a large number of such iontophoresis devices as described above have been proposed (see, for example, JP 63-35266 A, JP 04-297277 A, JP 2000-229128 A, JP 2000-229129 A, JP 2000-237327 A, JP 2000-237328 A, WO 03/037425 A1).

Such conventional iontophoresis device as described above qualifies, in principle, for the transdermal administration of a single drug. It should be noted that the administration of only one kind of sleep-inducing agent as a drug by means of an iontophoresis device may involve the emergence of a problem of poor awakening. The problem can be alleviated by administering a stimulant as another drug at the time of awakening.

Therefore, it is an important problem to enable the control of: the administered dose of each of two or more kinds of drugs, a sleep-inducing agent and a stimulant, to be administered; and the time at which each of the two or more kinds of drugs is administered by an iontophoresis device.

### SUMMARY OF THE INVENTION

The present invention has been made in view of the above-mentioned problems of the prior art, and an object of the present invention is to provide an iontophoresis device enabling the control of: the administered dose of each of a sleep-inducing agent and a stimulant to be administered; and the time at which each of them is administered.

To solve the above problems, the iontophoresis device of the present invention comprises: an electric power source device; a drug administration means comprising at least two or more electrode assemblies each comprising an ionic drug, the drug administration means being connected to the electric power source device; and a current control means for controlling a current flowing to the electrode assemblies individually, wherein the current flowing from the current control means allows the electrode assemblies to release the ionic drug in a predetermined amount at a predetermined time so that the ionic drug is administered transdermally to a living body, and wherein at least one of the two or more electrode assemblies comprising the ionic drug comprises a sleep-inducing agent as the ionic drug, and at least another one of the two or more electrode assemblies comprising the ionic drug comprises a stimulant as the ionic drug.

According to a preferred embodiment of the invention, the drug administration means comprises: two or more first electrode assemblies comprising the ionic drug; and one or more second electrode assemblies not comprising the ionic drug, the one or more second electrode assemblies serving as counter electrodes of the first electrode assemblies.

According to another preferred embodiment of the invention, the drug administration means comprises at least four or more electrode assemblies, the electrode assemblies comprising: one or more first electrode assemblies comprising the ionic drug; one or more second electrode assemblies comprising the ionic drug; one or more second electrode assemblies not comprising the ionic drug, which serve as counter electrodes of the one or more first electrode assemblies; and one or more first electrode assemblies not comprising the ionic drug, which serve as counter electrodes of the one or more second electrode assemblies.

Further, according to another preferred embodiment of the invention, the electrode assemblies comprising the ionic drug comprise at least : an electrode connected to an electric power source device having the same polarity as that of a drug component of the ionic drug in the electrode assemblies; an electrolyte solution holding portion impregnated with an electrolyte solution, the electrolyte solution holding portion being placed adjacent to the electrode; an ion exchange membrane which is selectively permeable to ions with a polarity opposite to that of a charged ion of the ionic drug, the ion exchange membrane being placed adjacent to the electrolyte solution holding portion; a drug solution holding portion impregnated with the ionic drug, the drug solution holding portion being placed adjacent to the ion exchange membrane; and an ion exchange membrane which is selectively permeable to an ion having the same polarity as that of the charged ion of the ionic drug, the ion exchange membrane being placed adjacent to the drug solution holding portion, and wherein the electrode assemblies not comprising the ionic drug comprise at least: an electrode having a polarity opposite to that of the electrode of the electrode assemblies comprising the ionic drug; an electrolyte solution holding portion impregnated with an electrolyte solution, the electrolyte solution holding portion being placed adjacent to the electrode; and an ion exchange membrane which is selectively permeable to an ion having a polarity opposite to that of the charged ion of the ionic drug, the ion exchange membrane being placed adjacent to the electrolyte solution holing portion.

Furthermore, according to another preferred embodiment of the invention, the electrode assemblies not comprising the ionic drug comprise at least : an electrode having a polarity opposite to that of the electrode in the electrode assemblies comprising the ionic drug; an electrolyte solution holding portion impregnated with an electrolyte solution, the electrolyte solution holding portion being placed adjacent to the electrode; an ion exchange membrane which is selectively permeable to an ion having the same polarity as that of the charged ion of the ionic drug, the ion exchange membrane being placed adjacent to the electrolyte solution holding portion; an electrolyte solution holding portion impregnated with an electrolyte solution, the electrolyte solution holding portion being placed adjacent to the ion exchange membrane; and an ion exchange membrane which is selectively permeable to an ion having a polarity opposite to that of the charged ion of the ionic drug, the ion exchange membrane being placed adjacent to the electrolyte solution holding portion.

Further, in another preferred embodiment of the invention, the drug administration means is configured integrally.

Furthermore, according to another preferred embodiment of the invention, the current control means comprises: a load resistor provided between the electrode assemblies and the electric power source device; a current detecting portion for detecting a current flowing to the load resistor; and a feedback control portion for allowing a current under the controlled condition to flow to the electrode assemblies in accordance with an output from the current detecting portion.

Furthermore, according to another preferred embodiment of the invention, the method of operating the iontophoresis device at least comprises: placing the drug administration means on a skin surface of a living body; energizing the iontophoresis device with the electric power source device; controlling a current flowing to the electrode assemblies individually by the current control means so as to allow a current under controlled condition to flow to the electrode assemblies; and allowing the electrode assemblies to release a sleep-inducing agent and a stimulant that are ionic drugs in predetermined amounts at a predetermined time.

As described above, in the iontophoresis device according to the present invention, current control means for individually controlling each of the currents flowing into multiple electrode assemblies separately comprising a sleep-inducing agent and a stimulant as ionic drugs is installed, and each of the electrode assemblies is adapted to release a predetermined amount of ionic drug at a predetermined time, that is, at a predetermined timing and for a predetermined time period in accordance with a current flowing from the current control means. As a result, each of the sleep-inducing agent and the stimulant can be administered while the administered dose and the timing thereof are controlled.

Figure 1 shows a bottom view of an iontophoresis device according to the present invention.
Fig. 2 shows a sectional view of drug administration means of the iontophoresis device according to the present invention.
Fig. 3 shows a circuit diagram of the iontophoresis device according to the present invention.

### DETAILED DESCRIPTION OF THE INVENTION

As described above, the iontophoresis device according to the present invention comprises: an electric power source device; a drug administration means comprising at least two or more electrode assemblies each comprising an ionic drug, the drug administration means being connected to the electric power source device; and a current control means for controlling a current flowing to the electrode assemblies individually, wherein the current flowing from the current control means allows the electrode assemblies to release the ionic drug in a predetermined amount at a predetermined time so that the ionic drug is administered transdermally to a living body, and wherein at least one of the two or more electrode assemblies comprising the ionic drug comprises a sleep-inducing agent as the ionic drug, and at least another one of the two or more electrode assemblies comprising the ionic drug comprises a stimulant as the ionic drug.

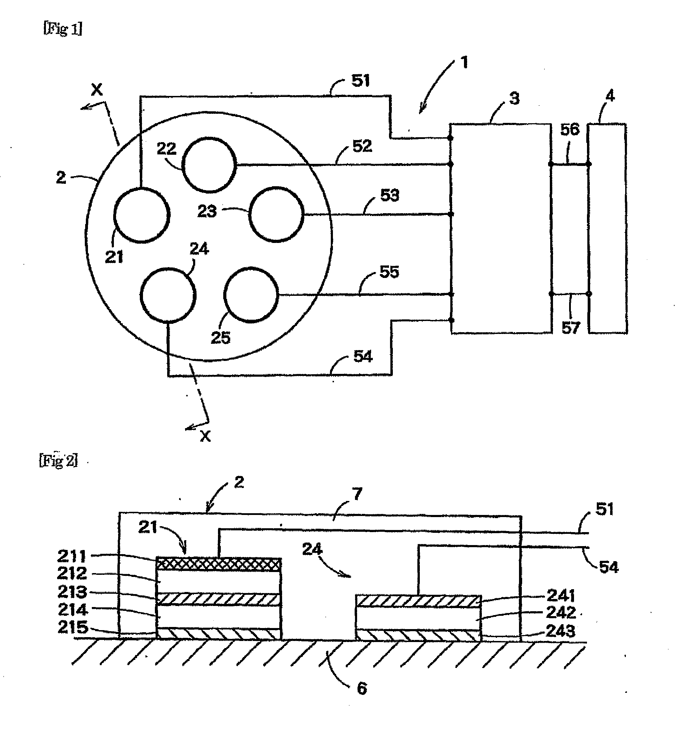

Hereinafter, the present invention is described on the basis of the preferred specific examples shown in the drawings. Fig. 1 is a bottom view of an iontophoresis device 1. The iontophoresis device 1 is constituted by: drug administration means 2 mounted on the skin of a living body; current control means 3; and an electric power source device 4. The drug administration means 1 includes multiple electrode assemblies (21, 22, 23, 24, and 25). In addition, first electrode assemblies (21, 22, and 23) out of the electrode assemblies in the drug administration means 2 are connected to the current control means 3 via electric wires (51, 52, and 53), and second electrode assemblies (24 and 25) as counter electrodes of the first electrode assemblies (21, 22, and 23) are connected to the current control means 3 via electric wires (54 and 55). Furthermore, the current control means 3 is connected to the electric power source device 4 via electric wires (56 and 57). Although not shown, only one second electrode assembly may be arranged. In that case, in Fig. 1, the second electrode assembly 25 and the electric wire 55 are deleted, and the second electrode assembly 24 is connected to the current control means 3 via the electric wire 54.

In the above specific example, the respective electrode assemblies (21, 22, 23, 24, and 25) in the drug administration means 2 are gathered in one package to be constituted integrally. They may be constituted so as to be apart from one another. Alternatively, only a part of the multiple electrode assemblies may be gathered in one package.

In the above specific example, the drug administration means 2, the current control means 3, and the electric power source device 4 are arranged at positions apart from one another. For example, the following procedure may be adopted: the electric power source device 4 is constituted as a button battery and the current control means 3 is constituted as an integrated circuit for achieving size reduction, thereby constituting the drug administration means 2, the current control means 3, and the electric power source device 4 integrally.

In the drug administration means of the present invention, in order to administer a sleep-inducing agent and a stimulant separately as a ionic drug, electrode assembly comprising the drug may have its own counter electrodes. Alternatively, only one counter electrode may be arranged when the sleep-inducing agent and the stimulant are ionized to have the same polarity.

Description will be given of the case where the first electrode assembly 21 comprises an ionic drug and the second electrode assembly 24 is a counter electrode not comprising an ionic drug in Fig. 2 by way of the specific constitution of an electrode assembly.

Fig. 2 is a sectional view of the drug administration means 2 shown in Fig. 1 taken along the line X-X'. In Fig. 2, the drug administration means 2 is arranged on a skin 6. In addition, the electrode assemblies 21 and 24 are backed by one package 7.

The first electrode assembly 21 comprises at least : an electrode 211 connected to the same polarity of the electric power source device 3 as that of a drug component of an ionic drug in the first electrode assembly 21 via the electric wire 51; an electrolyte solution holding portion 212 impregnated with the electrolyte solution, the electrolyte solution holding portion 212 being placed adjacent to the electrode 211; an ion exchange membrane 213 which is selectively permeable to an ion having polarity opposite to that of a charged ion of the ionic drug, the ion exchange membrane 213 being placed adjacent to the electrolyte solution holding portion 212; a drug solution holding portion 214 impregnated with the ionic drug, the drug solution holding portion 214 being placed adjacent to the ion exchange membrane 213; and an ion exchange membrane 215 which is selectively permeable to an ion having the same polarity as that of the charged ion of the ionic drug, the ion exchange membrane 215 being placed adjacent to the drug solution holding portion 214.
The second electrode assembly 24 connected to the electric power source device 3 via the electric wire 54 at least comprises: an electrode 241 opposite in polarity to the electrode 211 in the first electrode assembly 21; an electrolyte solution holding portion 242 impregnated with the electrolyte solution, the electrolyte solution holding portion 242 being placed adjacent to the electrode 241; and an ion exchange membrane 243 which is selectively permeable to an ion having polarity opposite to that of the charged ion of the ionic drug in the first electrode assembly 21, the ion exchange membrane 243 being placed adjacent to the electrolyte solution holding portion 242.

The second electrode assembly 24 is of the same electrode structure as that of the above-described first electrode assembly 21 when the second electrode assembly 24 comprises an ionic drug. Even when the second electrode assembly 24 does not comprise any ionic drug, the second electrode assembly 24 can be of the same electrode structure as that of the above-described first electrode assembly 21.

When the electrode assembly 21 comprising the ionic drug is energized, the ionic drug in the drug solution holding portion 214 moves owing to electrophoresis toward the side opposite to the electrode 211 by virtue of an electric field, and is administered into a skin via the ion exchange membrane 215. At this time, the ion exchange membrane 213 placed on the side of the electrode 211 selects an ion having polarity opposite to that of a charged ion of the ionic drug, thereby preventing the movement of the ionic drug toward the electrode 211. Meanwhile, the ion exchange membrane 215 placed on the skin selects an ion having the same polarity as that of the charged ion of the ionic drug, so the ionic drug is efficiently released, and can be administered into the skin at high transport efficiency. At the same time, an ion opposite in polarity to the ionic drug is prevented from moving from the side of a living body to the side of the drug solution holding portion 214. The movement of H⁺ or OH- generated at an electrode toward the skin is also suppressed, so a change in pH on the skin is suppressed. Furthermore, the electrode assembly in the present invention has such constitution as described above, thereby preventing damage to the skin based on an electrochemical reaction and enabling the ionic drug to be safely administered.

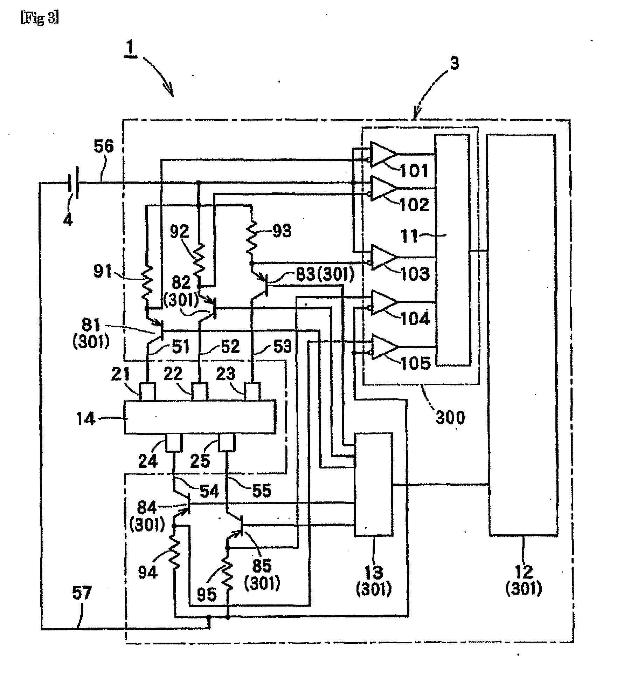

Next, a preferred specific example of the current control means of the iontophoresis device 1 will be described with reference to Fig. 3. The iontophoresis device 1 has such circuit as shown in Fig. 3, thereby enabling a predetermined amount of ionic drug to be released at a predetermined time and enabling control such that a current having a predetermined value flows into each electrode assembly comprising an ionic drug irrespective of the impedance and change with time of a skin.

As shown in Fig. 3, the current control means 3 in the iontophoresis device 1 comprises: load resistances (91, 92, 93, 94, and 95) provided between the electrode assemblies (21, 22, 23, 24, and 25) and the electric power source device 4; current detecting portions 300 (101, 102, 103, 104, 105, and 11) detecting currents flowing into the load resistances (91, 92, 93, 94, and 95); and feedback control portions 301 (12 and 13, and 81, 82, 83, 84, and 85) allowing a currents under the controlled condition to flow into the electrode assemblies (21, 22, 23, 24, and 25) in accordance with outputs from the current detecting portions 300 (101, 102, 103, 104, 105, and 11).

In addition, the current detecting portions 300 is constituted by: current detection circuits (101, 102, 103, 104, and 105) detecting currents flowing into the load resistances (91, 92, 93, 94, and 95); and an A/D converter 11 for converting an output from each of the current detection circuits (101, 102, 103, 104, and 105) into a digital signal, and which outputs the digital signal to each of the feedback control portions (12 and 13, and 81, 82, 83, 84, and 85).

In addition, the above-described feedback control portions 301 are constituted by: a CPU 12 that outputs a feedback signal to each of the electrode assemblies (21, 22, 23, 24, and 25) in accordance with an output from each of the current detecting portions (101, 102, 103, 104, 105, and 11); a D/A converter 13 for converting the feedback signal into an analog signal; and transistors (81, 82, 83, 84, and 85) placed between the electrode assemblies (21, 22, 23, 24, and 25) and the load resistances (91, 92, 93, 94, and 95) and allowing currents under controlled condition to flow into the electrode assemblies (21, 22, 23, 24, and 25) in accordance with outputs from the D/A converter 13. The emitters of the transistors (81, 82, 83, 84, and 85) are connected to the load resistances (91, 92, 93, 94, and 95), the bases of the transistors are connected to the D/A converter 13, and the collectors of the transistors are connected to the electrode assemblies (21, 22, 23, 24, and 25).

Differential amplifiers are preferably used for the current detection circuits (101, 102, 103, 104, and 105). Each of the differential amplifies detects a value for a voltage across each of the load resistances (91, 92, 93, 94, and 95). The amplifiers can detect current values from those voltage values and the resistance values of the above respective load resistances.

In addition, the load resistances (91, 92, 93, 94, and 95) are preferably fixed resistances. The resistance values in the fixed resistances can be appropriately set on the basis of, for example, a preset value for a current to flow into each of the electrode assemblies. The resistance values are each preferably about 10 Ω or less in consideration of, for example, an influence on the operating state of the iontophoresis device.

Next, the operation of the iontophoresis device 1 will be described with reference to Fig. 3.
At first, the current detection circuits (101, 102, 103, 104, and 105) detect currents flowing from the electric power source device 4 to the respective fixed resistances (91, 92, 93, 94, and 95). Signals in response to the detected currents are transmitted to the CPU 12 via the A/D converter 11. Next, the CPU 12 performs predetermined data processing in response to a signal from the A/D converter 11, and sends a feedback signal to the D/A converter 13. Next, the D/A converter 13 causes a current in response to the feedback signal from the CPU 12 to flow into a transistor. Then, in accordance with the currents flowing from the transistors (81, 82, 83, 84, and 85), a predetermined amount of ionic drug is released from each of the electrode assemblies (21, 22, and 23) at a predetermined time, so the ionic drug is transdermally administered to a living body 14.

The CPU 12 has a predetermined built-in algorithm, and performs data processing on the basis of the algorithm to output a feedback signal for causing each electrode assembly to release a predetermined amount of ionic drug at a predetermined time. Appropriately changing the program of the CPU in the above specific example can alter the order in which currents flow into the respective electrode assemblies, the time at which each of the currents flows, a combination of the respective electrode assemblies, and the like.

Furthermore, the CPU 12 can perform control such that a current having a predetermined value flows into each electrode assembly irrespective of the impedance and change with time of a skin. Such control can be performed in accordance with, for example, the following multivariate control.

Let I₉₁, I₉₂, I₉₃, I₉₄, and I₉₅ denote actual values for the currents in the respective load resistances (91, 92, 93, 94, and 95), and let V₉₁, V₉₂, V₉₃, V₉₄, and V₉₅ denote actual values for the voltages in the resistances. When a current vector Iᵢ = (I₉₁, I₉₂, I₉₃, I₉₄, I₉₅) and a voltage vector Vᵢ = (V₉₁, V₉₂, V₉₃, V₉₄, V₉₅) are defined, an expression (1) (Iᵢ = MA + MB x Vᵢ) is established. In the expression, MA denotes a matrix showing the internal state of a system independent of Vᵢ, and MB denotes a matrix showing each of a skin resistance and the internal resistance of an iontophoresis device against an ionic drug. In addition, MA and MB are estimated from Iᵢ and Vᵢ to be sequentially measured with the current detection circuits and from the expression (1). A control voltage Vᵢ for realizing a preset current value Iᵢ is calculated from the estimated MA and MB, and from an expression (2) (Vᵢ = Inv(MB) (Iᵢ - MA)) derived from the expression (1). The CPU 12 outputs a feedback signal for realizing the control voltage Vᵢ thus determined, and performs control in such a manner that a current having a predetermined value finally flows into each electrode assembly. Therefore, according to a preferred embodiment of the present invention, the current control means in the iontophoresis device performs control in such a manner that a current having a predetermined value flows into an electrode assembly.

In addition, the following conditions are adopted as preferred energizing conditions in the iontophoresis device 1.
(1) Constant current condition, specifically, 0.1 to 0.5 mA/cm², preferably 0.1 to 0.3 mA/cm²
(2) Safe voltage condition that realizes the above constant current, specifically, 50 V or less, preferably 30 V or less

In the present invention, the total number of electrode assemblies, and a combination of the number of first electrode assemblies and the number of second electrode assemblies are not limited to the above specific example, and the present invention can be embodied even when these numbers are appropriately changed. It is easy for one skilled in the art to conceive a constitution with those numbers appropriately changed from the above specific example. For example, the number of electrode assemblies can be increased or reduced by increasing or reducing the transistors, the fixed resistances, the current detection circuits, and the like shown in Fig. 3 by requested amounts.

In addition, the ionic drugs to be held by the respective electrode assemblies in the iontophoresis device of the present invention are a sleep-inducing agent and a stimulant. It should be noted that each of the sleep-inducing agent and the stimulant may be a combination of multiple drugs.

For the ionic drugs in the electrode assembly, examples of the sleep-inducing agent capable of being positively ionized include the following.
Narcotic: barbital, amobarbital, bromvalerylurea, rilmazafone hydrochloride, flunitrazepam, flurazepam hydrochloride, triazolam, midazolam, brotizolam, estazolam, lormetazepam, zopiclone, quazepam.
Tranquilizer: lithium carbonate, carbamazepine.
Antidepressant: nortriptyline hydrochloride, amoxapine, maprotiline hydrochloride, imipramine hydrochloride, amitriptyline hydrochloride, trimipramine maleate, clomipramine hydrochloride, lofepramine hydrochloride, dosulepin hydrochloride, trazodone hydrochloride, fluvoxamine maleate, paroxetine hydrochloride hydrate, milnacipran hydrochloride, mianserin hydrochloride, setiptiline maleate, tandospirone citrate.
Antianxiety: etizolam, clotiazepam, alprazolam, flutazolam, lorazepam, fludiazepam, bromazepam, mexazolam, diazepam, cloxazolam, chlordiazepoxide, prazepam, hydroxyzine.
Antihistaminic agent: diphenhydromine hydrochloride, diphenylpyraline hydrochloride, diphenylpyraline teoclate, clemastine fumarate, chlorpheniramine maleate, triprolidine hydrochloride, alimemazine tartrate, promethazine hydrochloride, homochlorcyclizine hydrochloride, cyproheptadine hydrochloride.

Meanwhile, examples of the sleep inducing agent capable of being negatively ionized include the following.
Narcotic: pentobarbital calcium, phenobarbital sodium, secibarbital sodium, chloral hydrate.
Antidepressant: clorazeptate dipotassium, ethyl loflazepate.

On the other hand, examples of the stimulant capable of being positively ionized include the following.
Analeptic drug: caffeine, methamphetamine hydrochloride, methylphenidate hydrochloride, pemoline, fursultiamine.
Antidepressant: tandospirone citrate.
Cerebral circulation activator: citicoline, adenosine triphosphate disodium, meclofenoxate hydrochloride, tiapride hydrochloride, ifenprodil tartrate, nicergoline, ibudilast, dihydroergotixine mesilate, nizofenone fumarate, fasudil hydrochloride, norepinephrine.

Furthermore, an example of the stimulant capable of being negatively ionized includes the following.
Cerebral circulation activator: gamma-aminobutyric acid, calcium hopantenate.

Examples of main side effects of a sleep-inducing agent include: poor awakening and dull feeling during the daytime due to the transfer of an effect of a narcotic up to the time of rising or any time after the rising; drift and weakness due to the muscle-relaxing effect possessed by the narcotic; and headache dull and malaise due to the transfer of an effect of the narcotic up to any time after the rising. Administering a stimulant upon or immediately before awakening can considerably alleviate those side effects.

In addition, an inactive electrode made of a conductive material such as carbon or platinum can be preferably used as the electrode of the electrode assembly. The electrolyte solution holding portion can be constituted by a thin film that has the property of holding an electrolyte solution by being impregnated with the electrolyte solution. The thin film can be made of the same material as that used for a drug solution holding portion used for holding an ionic drug by being impregnated with the ionic drug to be described later.

A desired one can be appropriately used as the electrolyte solution depending upon the conditions such as a drug to be applied. However, an electrolyte solution that damages the skin of a living body owing to an electrode reaction should be avoided. An organic acid or a salt thereof present in a metabolic cycle of a living body is preferable as the electrolyte solution in the present invention in terms of harmlessness. For example, lactic acid and fumaric acid are preferable. To be specific, an aqueous solution of 1M of lactic acid and 1M of sodium fumarate (1 : 1) is preferable. Such electrolyte solution is preferable because: it has high solubility with respect to water and passes a current well; and in the case where a current is allowed to flow at a constant level, the electric resistance is low and a change in pH is relatively small in an electric power source device.

A cation exchange membrane and an anion exchange membrane are preferably used together as ion exchange membranes to be used for an electrode assembly. Preferable examples of the cation exchange membrane include NEOSEPTAs (CM-1, CM-2, CMX, CMS, CMB, and CLE04-2) manufactured by Tokuyama Corporation. Preferable examples of the anion exchange membrane include NEOSEPTAs (AM-1, AM-3, AMX, AHA, ACH, ACS, ALE04-2, and AIP-21) manufactured by Tokuyama Corporation. Other preferable examples include: a cation exchange membrane that includes a porous film having cavities a part or whole of which are filled with an ion exchange resin having a cation exchange function; and an anion exchange resin that includes a porous film having cavities a part or whole of which are filled with an ion exchange resin having an anion exchange function.

The above-mentioned ion exchange resins can be fluorine-based ones that include a perfluorocarbon skeleton having an ion exchange group and hydrocarbon-based ones that include a nonfluorinated resin as a skeleton. From the viewpoint of convenience of production process, hydrocarbon-based ion exchange resins are preferably used. The filling rate of the porous film with the ion exchange resin, which varies depending on the porosity of the porous film, can be, for example, 5 to 95 % by mass, and is preferably 10 to 90 % by mass, or more preferably 20 to 60 % by mass.

The ion exchange group in the above-mentioned ion exchange resin is not particularly limited so far as it is a functional group that generates a group having negative or positive charge in aqueous solutions. Such functional group may be present in the form of a free acid or a salt. Examples of a cation exchange group include a sulfonic group, a carboxylic acid group, and a phosphonic acid group. Of those, a sulfonic group is preferable. Examples of a counter cation for the cation exchange group include: alkali cations such as a sodium ion and a potassium ion; and ammonium ions. Examples of an anion exchange group include a primary amino group, a secondary amino group, a tertiary amino group, a quaternary ammonium group, a pyridyl group, an imidazole group, a quaternary pyridium group, and a quaternary imidazolium group. Of those, a quaternary ammonium group or a quaternary pyridium group is preferable. Examples of a counter cation for the anion exchange group include: halogen ions such as a chlorine ion; and hydroxy ions.

The above-mentioned porous film is not particularly limited and any porous film can be used as far as it is in the form of a film or sheet that has a large number of pores communicating both sides thereof. To satisfy both of high strength and flexibility, it is preferable that the porous film be made of a thermoplastic resin. Examples of the thermoplastic resin constituting the porous film include: polyolefin resins such as homopolymers or copolymers of a-olefins such as ethylene, propylene, 1-butene, 1-pentene, 1-hexene, 3-methyl-1-butene, 4-methyl-1pentene, and 5-methyl-1-heptene; vinyl chloride-based resins such as polyvinyl chloride, vinyl chloride-vinyl acetate copolymers, vinyl chloride-vinylidene chloride copolymers, and vinyl chloride-olefin copolymers; fluorine-based resins such as polytetrafluoroethylene, polychlorotrifluoroethylene, polyvinylidene fluoride, tetrafluoroethylene-hexafluoropropylene copolymers, tetrafluoroethylene-perfluoroalkyl vinylether copolymers, and tetrafluoroethylene-ethylene copolymers; polyamide resins such as nylon 66; and polyimide resins. Of those, polyolefin resins are preferable in consideration of, for example, mechanical strength, flexibility, chemical stability, and chemical resistance. Of those, polyethylene or polypropylene is more preferable, and polyethylene is still more preferable.

The properties of the above-mentioned porous film made of the thermoplastic resin are not particularly limited. However, the mean pore size is preferably 0.005 to 5.0 µm, more preferably 0.01 to 2.0 µm, or still more preferably 0.02 to 0.2 µm in consideration of the formation of an ion exchange membrane that is thin and has excellent strength and low electric resistance. The above-mentioned mean pore size as used herein means a mean flow pore size measured in conformance with the bubble point method (JIS K3832-1990). Similarly, the porosity of the porous film is preferably 20 to 95 %, more preferably 30 to 90 %, or still more preferably 30 to 60 %. In consideration of the thickness of an ion exchange membrane to be finally formed, the thickness of the porous film is preferably 5 to 140 µm, more preferably 10 to 130 µm, or still more preferably 15 to 55 µm. An anion exchange membrane or a cation exchange membrane formed of such porous film generally has the same thickness as that of the porous film or up to about 20 µm larger than the thickness of the porous film.

Furthermore, the drug solution holding portion is constituted by a thin film that holds an ionic drug by being impregnated with the ionic drug or the like. It is important for such thin film to have a sufficient ability of holding an ionic drug by being impregnated with the ionic drug or the like, and a sufficient ability (ion transferability, ion conductivity) of moving an ionized drug impregnated into and held by the thin film to the skin side under predetermined electric field conditions. Examples of a material that brings together good property of holding a drug by being impregnated with the drug and good ion conductivity include hydrogel forms of acrylic resins (acrylic hydrogel film), a segmented polyurethane-based gel film, and an ion-conductive porous sheet for forming a gel-like solid electrolyte (such as a porous polymer disclosed in JP 11-273452 A using, as a base, an acrylonitrile copolymer containing 50 mol% or more, preferably 70 to 98 mol% or more of acrylonitrile and having a porosity of 20 to 80%). When such drug solution holding portion as described above is impregnated with a drug, an impregnation rate (defined by 100×(W-D)/D [%] where D indicates a dry weight and W indicates a weight after impregnation) is preferably 30 to 40%.

When multiple electrode assemblies are gathered in one package so that drug administration means is constituted integrally, any material can be used for the package without any particular limitation as long as the material does not affect the administration of an ionic drug. For example, polyolefin for medical equipment, and the like is available.

Patent Document 7 according to the applicant of the present invention describes details about the above-mentioned respective components, and the contents described in the document are also included in the present invention.

## Claims

1. An iontophoresis device comprising:
an electric power source device;
a drug administration means comprising at least two or more electrode assemblies each comprising an ionic drug, the drug administration means being connected to the electric power source device; and
a current control means for controlling a current flowing to the electrode assemblies individually,
wherein the current flowing from the current control means allows the electrode assemblies to release the ionic drug in a predetermined amount at a predetermined time so that the ionic drug is administered transdermally to a living body, and
wherein at least one of the two or more electrode assemblies comprising the ionic drug comprises a sleep-inducing agent as the ionic drug, and at least another one of the two or more electrode assemblies comprising the ionic drug comprises a stimulant as the ionic drug.

2. The iontophoresis device according to claim 1, wherein the drug administration means comprises:
two or more first electrode assemblies comprising the ionic drug; and
one or more second electrode assemblies not comprising the ionic drug, the one or more second electrode assemblies serving as counter electrodes of the first electrode assemblies.

3. The iontophoresis device according to claim 1, wherein the drug administration means comprises at least four or more electrode assemblies, the electrode assemblies comprising:
one or more first electrode assemblies comprising the ionic drug;
one or more second electrode assemblies comprising the ionic drug;
one or more second electrode assemblies not comprising the ionic drug, which serve as counter electrodes of the one or more first electrode assemblies; and
one or more first electrode assemblies not comprising the ionic drug, which serve as counter electrodes of the one or more second electrode assemblies.

4. The iontophoresis device according to claim 1, wherein the electrode assemblies comprising the ionic drug comprise at least:
an electrode connected to an electric power source device having the same polarity as that of a drug component of the ionic drug in the electrode assemblies;
an electrolyte solution holding portion impregnated with an electrolyte solution, the electrolyte solution holding portion being placed adjacent to the electrode;
an ion exchange membrane which is selectively permeable to ions with a polarity opposite to that of a charged ion of the ionic drug, the ion exchange membrane being placed adjacent to the electrolyte solution holding portion;
a drug solution holding portion impregnated with the ionic drug, the drug solution holding portion being placed adjacent to the ion exchange membrane; and
an ion exchange membrane which is selectively permeable to an ion having the same polarity as that of the charged ion of the ionic drug, the ion exchange membrane being placed adjacent to the drug solution holding portion, and
wherein the electrode assemblies not comprising the ionic drug comprise at least:
an electrode having a polarity opposite to that of the electrode of the electrode assemblies comprising the ionic drug;
an electrolyte solution holding portion impregnated with an electrolyte solution, the electrolyte solution holding portion being placed adjacent to the electrode; and
an ion exchange membrane which is selectively permeable to an ion having a polarity opposite to that of the charged ion of the ionic drug, the ion exchange membrane being placed adjacent to the electrolyte solution holing portion.

5. The iontophoresis device according to claim 4, wherein the electrode assemblies not comprising the ionic drug comprise at least :
an electrode having a polarity opposite to that of the electrode in the electrode assemblies comprising the ionic drug;
an electrolyte solution holding portion impregnated with an electrolyte solution, the electrolyte solution holding portion being placed adjacent to the electrode;
an ion exchange membrane which is selectively permeable to an ion having the same polarity as that of the charged ion of the ionic drug, the ion exchange membrane being placed adjacent to the electrolyte solution holding portion;
an electrolyte solution holding portion impregnated with an electrolyte solution, the electrolyte solution holding portion being placed adjacent to the ion exchange membrane; and
an ion exchange membrane which is selectively permeable to an ion having a polarity opposite to that of the charged ion of the ionic drug, the ion exchange membrane being placed adjacent to the electrolyte solution holding portion.

6. The iontophoresis device according to claim 1, wherein the drug administration means is configured integrally.

7. The iontophoresis device according to claim 1, wherein the current control means comprises:
a load resistor provided between the electrode assemblies and the electric power source device;
a current detecting portion for detecting a current flowing to the load resistor; and
a feedback control portion for allowing a current under the controlled condition to flow to the electrode assemblies in accordance with an output from the current detecting portion.

8. A method of operating the iontophoresis device according to claim 1, comprising at least:
placing the drug administration means on a skin surface of a living body;
energizing the iontophoresis device with the electric power source device;
controlling a current flowing to the electrode assemblies individually by the current control means so as to allow a current under controlled condition to flow to the electrode assemblies; and
allowing the electrode assemblies to release a sleep-inducing agent and a stimulant that are ionic drugs in predetermined amounts at a predetermined time.
